# EUROPÄISCHE PATENTSCHRIFT

(11) **EP 2 693 943 B1**
(45) Veröffentlichungstag und Bekanntmachung des Hinweises auf die Patenterteilung: **01.09.2021**
(21) Anmeldenummer: 12712966.6
(22) Anmeldetag: 02.04.2012
(51) Int. Cl.: A61B 5/1455, A61B 5/1468, A61B 5/145, G01N 33/49

(54) **ANALYSESYSTEM MIT MESSVORRICHTUNG UND TESTELEMENT**
ANALYSIS SYSTEM HAVING A MEASUREMENT DEVICE AND A TESTING ELEMENT
SYSTÈME D'ANALYSE AVEC DISPOSITIF DE MESURE ET ÉLÉMENT D'ESSAI

(30) Priorität: 05.04.2011 EP 11002813
(43) Veröffentlichungstag der Anmeldung: 12.02.2014
(73) Patentinhaber: Roche Diabetes Care GmbH, 68305 Mannheim (DE); F. Hoffmann-La Roche AG, 4070 Basel (CH)
(72) Erfinder: EIKMEIER, Heino, 64653 Lorsch (DE); HAAR, Hans-Peter, 69168 Wiesloch (DE); HOENES, Joachim, 64673 Zwingenberg (DE); HORN, Carina, 68647 Biblis (DE); RIEGER, Ewald, 67240 Bobenheim-Roxheim (DE)
(74) Vertreter: Pfiz, Thomas
(86) Internationale Anmeldenummer: PCT/EP2012/001476
(87) Internationale Veröffentlichungsnummer: WO 2012/136352

(56) Entgegenhaltungen:
- EP-A1- 1 424 040
- EP-A1- 2 116 180
- EP-A2- 1 048 310
- WO-A1-00/40150
- WO-A2-2007/122380
- US-A1- 2010 150 777
- US-A1- 2010 331 653
- US-A1- 2014 329 258

## Beschreibung

Die Erfindung betrifft eine tragbare diagnostische Messvorrichtung zur Bestimmung mindestens eines Analyseparameters einer Körperflüssigkeit, insbesondere zur Bestimmung einer Analytkonzentration in einer Körperflüssigkeit und besonders bevorzugt für Blutglukosewertbestimmungen, sowie ein Analysesystem, das die Messvorrichtung und mindestens ein disposibles (einmalig zu verwendendes) Testelement umfasst. Derartige Analysesysteme sind im Stand der Technik unter anderem aus US2014/329258 beziehungsweise EP2248590 bekannt.

Bei Teststreifen für Blutglukosebestimmungen wurden in der Vergangenheit verschiedene Applikationsformen für den Probenauftrag relativ zu der flach-rechteckförmigen Streifengeometrie realisiert, speziell auch das so genannte Top-dosing, also der Blutauftrag von oben auf das flachseitig zwischen den beidseitigen Schmalseiten der Messvorrichtung aufgebrachte Testfeld. Bei optisch arbeitenden Systemen erfolgt die Vermessung des Testfelds üblicherweise durch ein Loch in der Trägerfolie mittels in Reflexion gemessener Absorptionsphotometrie.

Bei optischen Messungen muss der Teststreifen über der optischen Messeinheit positioniert werden und das Blut auf den Teststreifen gegeben werden. Bei auf dem Markt bereitgehaltenen Top-dosing-Systemen wird der Teststreifen zur Beaufschlagung mit Blut jedoch inmitten einer Gehäusewanne und damit innerhalb der Gerätekontur positioniert. Das bedeutet, der Nutzer muss das Blut an seinem Finger auf den Teststreifen inmitten des Geräts platzieren. Dabei hat er einerseits Schwierigkeiten den Zielort hinter seinem Finger zu sehen. Andererseits kann es leicht passieren - speziell, wenn relativ viel Blut am Finger hängt - dass Blut auf das Gerät oder sogar an oder in die Öffnung zur optischen Messeinheit läuft. Durch ungenaues Applizieren bzw. zu große Probemengen kann daher die Wanne des Teststreifenhalters oder die optische Messeinheit verunreinigt werden.

Hingegen wird beim so genannten Outside-dosing der Streifen entnommen, außerhalb des Geräts Probe aufgetragen und der Streifen dann zurück ins Gerät gesteckt. Wie bei Top-dosing-Modellen kann es zur Verschmutzung von Oberflächen kommen, da die komplette Streifenfläche zur Applikation nutzbar ist. Zusätzlich kann aber auch eine ungewollte Verschmutzung der Streifenunterseite auftreten, beispielsweise wenn der Streifen für die Probenapplikation auf einer verschmutzten Oberfläche abgelegt wird. Derartige Verschmutzungen können eine Fehlmessung zur Folge haben.

Bei Out-of-meter-dosing-Systemen wird Blut durch Kapillaren des Teststreifens zum Messort transportiert. Die benötigte Blutmenge ist jedoch wesentlich größer als bei Methoden, bei denen das Testfeld direkt mit Blut beaufschlagt wird, weil zunächst die Kapillaren mit Blut gefüllt werden müssen. Dieser Nachteil kann zwar durch elektrochemisch arbeitende Teststreifen vermieden werden, indem durch Nach-außen-Führung der Elektroden Messungen mit üblichen Blutvolumina durchgeführt werden können. Die Herstellung solcher Kapillar-Teststreifen ist jedoch wegen der aufwändigen Montage vergleichsweise kompliziert und die Kosten auch wegen der höheren Materialkosten für Boden- und Deckfolie entsprechend hoch.

Dementsprechend lag der Erfindung die Aufgabe zugrunde, ein Analysesystem bereitzustellen, das die Möglichkeit bietet, mit kostengünstigen Testelementen eine vereinfachte Blutauftragung zu ermöglichen.

Die der Erfindung zugrunde liegende Aufgabe wurde durch die erfindungsgemäße Messvorrichtung und das erfindungsgemäße Analysesystem gemäß Anspruch 1 gelöst. Auf die in den Unteransprüchen wiedergegebenen bevorzugten Ausgestaltungen der Erfindung wird ausdrücklich Bezug genommen. Die erfindungsgemäße Messvorrichtung umfasst ein Gehäuse und eine Aufnahmefläche zur Aufnahme eines Testelements, das als Trägerstreifen ausgestaltet ist, wobei die Aufnahmefläche auf einer Schmalseite des Gehäuses angeordnet ist, sodass das Testelement flächig an der Aufnahmefläche anliegt. Das Testelement ist ein disposibles Testelement. Das erfindungsgemäße Analysesystem umfasst die Messvorrichtung und mindestens ein zur einmaligen Verwendung darin vorgesehenes Testelement, das als Trägerstreifen ausgestaltet ist.

Das Gehäuse der Messvorrichtung weist im Wesentlichen die geometrische Form eines Zylinders mit einer Grundfläche und einer Deckfläche auf, bevorzugt einem geraden Zylinder. Die Grundfläche bildet die Rückseite, die Deckfläche die Frontseite des Gehäuses. Die Grund- und die Deckfläche haben im Wesentlichen die Formen eines Polygons, z. B. eines Dreiecks eines Vierecks oder eines Fünfecks, einer Ellipse oder einer Kombination aus diesen. Die Mantelfläche des Zylinders ist als Schmalseiten ausgebildet. Haben Grund- und Deckfläche im Wesentlichen die Form eines Fünfecks, so hat das Gehäuse fünf Schmalseiten. Benachbarte Schmalseiten sind durch die Kanten, die z. B. die Ecken eines Polygons oder in einer anderen Ausführungsform die Übergangsecken vom Polygon zu Ellipse der Grundfläche mit der Deckfläche verbinden, voneinander getrennt. Diese Kanten können abgerundet sein.

Die Kanten der Schmalseiten mit der Grund- und/oder Deckfläche können ebenfalls abgerundet sein. In einer Ausgestaltung sind diese beiden Kanten an einer oder mehreren Schmalseiten derart abgerundet, dass sie zu einer neuen Kante vereinigt sind, oder einen kantenlosen Übergang von der Grundfläche zur Deckfläche bilden.

Die Grundfläche und/oder die Deckfläche können unabhängig voneinander eine konvexe oder konkave Krümmung aufweisen. Ebenso kann die Schmalseite, auf der die Aufnahmefläche angeordnet ist, gegenüber der Ebene, die die Aufnahmefläche aufspannt, gekrümmt sein. Bei elliptischer Grundfläche und konvex gekrümmter Grund- und Deckfläche mit abgerundeten Kanten weist das Gehäuse eine Eiform auf. Bevorzugt ist die Grundfläche konkav und die Deckfläche parallel hierzu konvex gekrümmt.

In einer bevorzugten Ausführungsform bildet eine gesamte Schmalseite die Aufnahmefläche. In einer anderen Ausführungsform bildet ein Abschnitt einer Schmalseite die Aufnahmefläche. Die Schmalseite oder der Abschnitt der Schmalseite, die die Aufnahmefläche bildet, ist - von einer optionalen Abdeckung insbesondere der Aufnahmefläche abgesehen - bevorzugt gegenüber benachbarten Bauteilen der Messvorrichtung erhaben und/oder weist eine exponierte Lage auf.

Die erfindungsgemäßen Messvorrichtungen können in medizinischen Labors eingesetzt werden. Die Erfindung richtet sich jedoch insbesondere auf Anwendungsfälle, bei denen die Analyse durch den Patienten selbst durchgeführt wird, um seinen Gesundheitszustand laufend zu überwachen ("home-monitoring"). Für derartige Zwecke kommt es besonders auf eine einfache Handhabung an, weil nur dadurch sichergestellt werden kann, dass die erforderlichen Analysen von den Patienten regelmäßig durchgeführt werden und die Genauigkeit des analytischen Ergebnisses nicht durch Handhabungsfehler beeinträchtigt wird. Außerdem müssen die Messvorrichtungen möglichst klein, leicht und robust sein. Daneben ist die Erfindung auch für die sogenannte patientennahe Diagnostik ("near patient testing") in besonderem Maße geeignet. Die Erfindung betrifft daher bevorzugt Handgeräte und/oder mobile Tischgeräte für die Analyse von Körperflüssigkeiten.

In einer bevorzugten Ausführungsform ist die Grundfläche und/oder die Deckfläche derart gewählt, dass die Messvorrichtung während der Messung bequem in einer Hand gehalten werden kann. Die Kanten können abgerundet sein, wodurch eine ergonomische Form des Gehäuses erhalten wird. Tischgeräte weisen bevorzugt Einrichtungen auf, die ein Wegrutschen auf glatten Oberflächen verhindern, beispielsweise Antirutschnoppen.

Insbesondere bei mobilen Tischgeräten kann derjenige Gehäuseteil, auf dem die Aufnahmefläche angeordnet ist, gegenüber dem übrigen Gehäuse derart abgewinkelt sein, dass er in Richtung des Nutzers zeigt und eine bequeme Beaufschlagung z. B. mit Blut erlaubt.

Die Hauptgeräteachse der Messvorrichtung liegt bevorzugt in der Geräteebene, die im Wesentlichen parallel zu der Grund- und Deckfläche des Gehäuses ist. Die Hauptgeräteachse ist eine gedachte Gerade durch die Messvorrichtung, die als Gestaltungs- und Ordnungsmittel genutzt wird, um die Ausrichtung und Lage ihrer Strukturen zu beschreiben. Sie gliedert die Frontseite der Messvorrichtung in zwei Hälften. Für den Fall, dass der Gehäuseteil, auf dem die Aufnahmefläche angeordnet ist, gegenüber dem übrigen Gehäuse abgewinkelt ist, weist die Messvorrichtung eine zweite Geräteachse auf. Die zweite Geräteachse liegt bevorzugt in der gleichen, die Frontseite der Messvorrichtung gliedernde Ebene wie die Hauptgeräteachse und schließt mit der Geräteebene eine Winkel von 20° bis 90°, bevorzugt 30° bis 70°, ebenfalls bevorzugt 45° bis 60° ein. In einer anderen Ausführungsform liegt die zweite Geräteachse im Wesentlichen senkrecht zu der die Frontseite der Messvorrichtung gliedernde Ebene. Der Ausdruck "im Wesentlichen senkrecht" bedeutet im Zusammenhang mit dieser Erfindung, dass ein Winkel im Bereich von 70° bis 110°, bevorzugt im Bereich von 80° bis 100°, besonders bevorzugt von 90° eingeschlossen wird. Gleichermaßen bedeutet der Ausdruck "im Wesentlichen parallel", dass ein Winkel im Bereich von -20° bis 20°, bevorzugt im Bereich von -10° bis 10° eingeschlossen wird. Besonders bevorzugt bedeutet er parallel.

In einer bevorzugten Ausführungsform ist die Längsachse der Aufnahmefläche (auch Achse der Aufnahmefläche genannt) im Wesentlichen senkrecht zu der die Frontseite der Messvorrichtung gliedernde Ebene, in der die Hauptgeräteachse liegt. Bei Handgeräten ist die Achse der Aufnahmefläche bevorzugt im Wesentlichen senkrecht zu der Hauptgeräteachse. Ist die erfindungsgemäße Messvorrichtung als mobiles Tischgerät ausgebildet, ist die Achse der Aufnahmefläche bevorzugt im Wesentlichen senkrecht zu der zweiten Geräteachse, die in der die Frontseite der Messvorrichtung gliedernde Ebene liegt. In einer anderen Ausführungsform, in der die zweite Geräteachse senkrecht zu der die Frontseite der Messvorrichtung gliedernde Ebene liegt, ist die Achse der Aufnahmefläche im Wesentlichen parallel zu der Hauptgeräteachse.

Die Ebene, die durch die Aufnahmefläche aufgespannt wird, kann senkrecht zu der Geräteebene sein. Die Geräteebene ist im Wesentlichen parallel zu den Ebenen, in den die Grund- und die Deckfläche des Gehäuses liegen. Sie ist senkrecht zu der Ebene, die die Frontseite der Messvorrichtung gliedert.

In einer anderen Ausführungsform ist die Ebene, die durch die Aufnahmefläche aufgespannt wird, um die Achse der Aufnahmefläche gedreht. Bevorzugt ist ein Drehwinkel im Bereich von - 35° bis 90°. Der Drehwinkel beschreibt die Drehung der Ebene, die durch die Aufnahmefläche aufgespannt wird, um die Längsachse der Aufnahmefläche bezogen auf eine Fläche, die senkrecht zur Geräteebene ist. Ein positives Vorzeichen gibt hier die Drehung in Richtung der Deckfläche, ein negatives Vorzeichen die Drehung in Richtung der Grundfläche an. Der Drehwinkel entspricht in einer Ausführungsform für eine Messvorrichtung, bei der der Gehäuseteil, auf dem die Aufnahmefläche angeordnet ist, gegenüber dem übrigen Gehäuse derart abgewinkelt ist, 90°. Die Aufnahmefläche ist also parallel zur Front- und Rückseite der Messvorrichtung. In einer weiteren besonderen Ausgestaltung der Erfindung mit abgewinkeltem Gehäuseteil liegt der Drehwinkel im Bereich von 20° bis 70°, bevorzugt im Bereich von 55° bis 65°. In einer ebenfalls bevorzugten Ausführungsform ohne abgewinkeltem Gehäuseteil liegt der Drehwinkel im Bereich von 0° bis -30°, bevorzugt im Bereich von -10° bis -25°.

Um eine Verschmutzung durch aufgetragenes Blut erfolgreicher zu vermeiden, ist in einer bevorzugten Ausführungsform die gesamte Aufnahmefläche oder ein Abschnitt der Aufnahmefläche, bevorzugt zumindest der Abschnitt der Aufnahmefläche im Testfeldbereich, schmaler ausgebildet als die vorgesehenen Testelemente in den entsprechenden Abschnitten, so dass ein Testelement, das in der Messposition positioniert ist, die Aufnahmefläche, insbesondere im Bereich des Testfeldes überdeckt.

Die Messvorrichtung umfasst bevorzugt eine Messeinheit zum Messen einer Messgröße an einem in einer Messposition (auch Positionierungsendlage genannt) positionierten Testelement. Die Aufnahmefläche weist eine Messöffnung auf, so dass der Testfeldbereich eines in der Messposition befindlichen Testelements auf der Messöffnung aufliegt. Bevorzugt liegt das Testelement derart auf, dass sein Testfeldbereich einen definierten Abstand von einer unterhalb der Messöffnung und damit im Geräteinnern befindlichen optischen Messeinheit hat. In einer anderen Ausführungsform weist die Messeinheit Gerätekontakte auf, die mit Sensorkontakten eines elektrochemisch arbeitenden Testelements, das sich in der Messposition befindet, in Kontakt stehen kann.

Die gemessenen Werte der Messgröße werden in einer bevorzugten Ausführungsform an eine Auswerteeinheit zur Ermittlung von Analysedaten aus gemessenen Werten der Messgröße übermittelt.

Insbesondere ist die Messvorrichtung zur Bestimmung der Glukosekonzentration im Blut geeignet. Andere wichtige Analyten sind Cholesterin und verschiedene Blutgerinnungsparameter. Das heißt, als Analyseparameter im Sinne der Erfindung ist nicht notwendigerweise die Konzentration einer Substanz in der Probenflüssigkeit zu verstehen, sondern die Erfindung bezieht sich auch auf andere (insbesondere auf medizinischem Gebiet) relevante Analyseparameter, wie hier die Blutgerinnungszeit. Die Erfindung ist hinsichtlich des Analyseparameters nicht beschränkt. Als Tragschicht bekannter Testelemente, auch Biosensoren genannt, werden meist längliche Kunststoffstreifen verwendet. Jedoch sind auch andere Formen geeignet, beispielsweise näherungsweise quadratische Plättchen.

Das disposible Testelement ist als Trägerstreifen ausgestaltet. Üblicherweise weist mindestens ein auf dem Trägerstreifen angeordnetes Testfeld eine Fläche zum Auftrag der Körperflüssigkeit auf. Befindet sich das Testelement in Messposition auf der Aufnahmefläche der Messvorrichtung, ist dessen Testfeld von der freien Oberseite her mit einer Körperflüssigkeit beaufschlagbar. Von der Unterseite kann bei optisch arbeitenden Testelementen eine Farbänderung registriert werden. Bei elektrochemisch arbeitenden Testelementen kann ein Strom gemessen werden.

Bevorzugt ist das Testelement ein so genannter "nicht abzuwischender" (non wipe) Teststreifen. Bei diesen Teststreifen durchläuft die Körperflüssigkeit, nachdem sie auf der Oberseite des Testfeldes aufgebracht ist, die gesamte Dicke des aus mehreren Schichten bestehenden Testfeldes. Dabei finden chemische Reaktionen zwischen der Körperflüssigkeit und den in dem Testfeld enthaltenen Reagenzien statt. Eine resultierende optisch nachweisbare Veränderung in einer Detektionsschicht kann von der Unterseite des Testelements her reflexionsphotometrisch detektiert werden. Die Basisschicht des Testelements hat im Bereich des Testfeldes zu diesem Zweck eine Ausnehmung.

Das Testelement kann in seiner Längsrichtung in drei Abschnitte unterteilt werden. Das Testfeld definiert einen Testfeldbereich. Bei Testelementen, die nur ein Testfeld aufweisen, wird der Testfeldbereich durch die vordere Kante und hintere Kante des Testfeldes begrenzt. Auf einem Testelement können in einem größeren Testfeldbereich auch mehrere Testfelder hintereinander angeordnet sein. Der Testfeldbereich erstreckt sich in diesem Fall in Einführrichtung von der vorderen Kante des ersten Testfeldes bis zu der hinteren Kante des letzten Testfeldes. Der Abschnitt zwischen dem Vorderende (mit welchem das Testelement z. B. in eine Halterung der Aufnahmefläche eingeführt werden kann) und dem Testfeldbereich wird als Vorderabschnitt bezeichnet. Zwischen dem (dem Vorderende gegenüberliegenden) Handhabungsende des Testelements und dem Testfeldbereich erstreckt sich ein Handhabungsabschnitt. Das Testelement weist bevorzugt im Vorderabschnitt in der Nähe des Vorderendes eine Ausnehmung auf, die in Querrichtung des Testelements mittig angeordnet ist.

In einer weiteren Ausführungsform weist die Messvorrichtung eine Heizeinrichtung zum Erwärmen des Testfelds eines in der Messposition positionierten Testelements auf. Die Messvorrichtung kann eine Temperaturmesseinrichtung zur Bestimmung der Temperatur des Testfeldes eines in der Messposition positionierten Testelements aufweisen. Mittels einer optionalen Thermostatisierungselektronik lässt sich das Testfeld mit Hilfe der Temperaturmesseinrichtung und der Heizeinrichtung auf eine gewünschte Solltemperatur thermostatisieren.

Für die exakte Positionierung der Testelemente beispielsweise gegenüber dem Reflexionsphotometer bei optischer Messung oder den Kontakten bei elektrochemischer Messung sind in den Messvorrichtungen Positioniereinrichtungen vorhanden. Die Genauigkeit der Messung und die Einfachheit der Handhabung werden wesentlich durch die Positioniereinrichtung bestimmt. Die Positionierung des Testelements auf der Aufnahmefläche bezieht sich auf alle drei Raumrichtungen, nämlich auf die Längs- und Querrichtung des Testfeldes und auf die Richtung vertikal zu der Testfeldoberfläche. Die Aufnahmefläche weist bevorzugt wenigstens eine Einrichtung zum Positionieren des Testelements auf.

Der vertikale Abstand der Oberfläche des Testfeldes von der Messoptik ist ein entscheidender Parameter für eine exakte Messung. Bevorzugt wird der vertikale Abstand festgelegt, in dem das Testelement mit seiner Unterseite flach an der Aufnahmefläche anliegt.

Da aus Kostengründen in zunehmendem Maße dazu übergegangen wird, die Testfeldflächen immer kleiner zu machen, muss auch die Längs- und Querpositionierung der Testelemente sehr exakt vorgenommen werden, um einen möglichst großen Teil der Oberfläche des Testfelds als Messfläche verwenden zu können. Eine falsche räumliche Ausrichtung der Testelemente führt unmittelbar zu einer Verkleinerung der effektiven Messfläche und dadurch zu einem Messfehler. In einer Ausführungsform weist die Aufnahmefläche eine längsseitige Führung auf, um beim Einführungsvorgang dem Testelement eine Zwangsrichtung vorzugeben. Die Führung dient ebenfalls zur Querpositionierung in der Messposition. Die Führungselemente können einseitig oder beidseitig des in der Messposition positionierten Testelements angeordnet sein. In einer Ausführungsform sind Führungselemente im Bereich der Messöffnung beidseitig des in der Messposition positionierten Testelements angeordnet.

Bevorzugt weisen die Führungselemente die Form von Schienen auf, die eine Links-rechts-Verschiebung des Testelements verhindern. In einer bevorzugten Ausgestaltung der Erfindung überdecken die Führungsschienen das Testelement seitlich vor und hinter dem Testfeld, um eine vertikale Verschiebung zu verhindern. In einer alternativen Ausführungsform weist die Aufnahmefläche Führungselemente im Bereich des Vorderendes und/oder im Bereich des Handhabungsendes des in der Messposition positionierten Testelements auf. In einer Ausgestaltung sind die Führungselemente beidseitig angeordnet, so dass nur eine Positionierung des Testelements längs der Aufnahmefläche möglich ist. Bei einer beidseitigen Anordnung können die links und rechts angeordneten Führungselemente integral geformt sein und so die Aufnahmefläche überdecken. Dies ist besonders im Bereich des Vorderabschnitts bevorzugt. In einer anderen Ausgestaltung sind Führungselemente im Bereich des Vorderendes und/oder im Bereich des Handhabungsendes des in der Messposition positionierten Testelements einseitig angeordnet. Hier kann der Positionierungsvorgang des Testelements quer zur Aufnahmefläche erfolgen.

In einer alternativen Ausgestaltung der Erfindung weist die Aufnahmefläche eine Halterung auf. Die Halterung kann unter einer Klappe mit Haltefedern befinden, die von oben auf den Teststreifen drücken.

Zur Positionierung kann das Testelement im Bereich seines Vorderendes eine Ausnehmung aufweisen. Die Positioniereinrichtung der Aufnahmefläche besitzt einen drehbar gelagerten konischen Nocken, der beim Einführen des Testelements in die Messvorrichtung in dessen Ausnehmung geschwenkt wird. In der Positionierungsendlage stößt das Testelement mit seinem Vorderende an einen Anschlag an und der schwenkbare konische Nocken drückt ihn auf eine Auflagefläche nieder. Dabei greift der Nocken in die Ausnehmung derart ein, dass das Testelement in allen drei Raumrichtungen kraftbelastet ist, wodurch eine Positionierung erreicht wird.

In einer weiteren Ausführungsform wird ein Testelement mit jeweils einer Ausnehmung an seinem Handhabungs- und Vorderende verwendet, in die jeweils ein Spannstift der Aufnahmefläche eingreift. Beim Positioniervorgang wird zunächst der Spannstift am Vorderende in die entsprechende Ausnehmung bewegt. Danach wird durch die Betätigung einer verschiebbaren Klappe das Testelement gebogen, so dass der sich im Bereich des Handhabungsendes befindende Spannstift in die zweite Ausnehmung eingreift. An den zweiten Spannstift greift eine Feder derart an, dass das Testelement in Längsrichtung unter Zugspannung steht. Durch diese Zugspannung wird das Testelement mit seiner Unterseite gegen eine Andruckplatte auf der Aufnahmefläche gedrückt, wodurch das auf der Oberseite des Testelements angeordnete Testfeld in der gewünschten Position ist.

In einer anderen Ausführungsform weist die Aufnahmefläche eine Führungselemente aufweisende Halterung auf, durch die ein Testelement beim Einführen in die Halterung in seiner Querrichtung geführt wird und die Unterseite eines in der Messposition befindlichen Testelements in seinem Testfeldbereich auf einer eine Messöffnung enthaltenden Aufnahmefläche derartig aufliegt, dass sein Testfeldbereich einen definierten Abstand von einer unterhalb der Messöffnung befindlichen Messeinheit hat, bei welchem die Halterung eine gegen die Unterseite des Teststreifens vorspringende und folglich gegenüber der Mittelebene des Testfeldbereiches in der Höhe (in Richtung von der Aufnahmefläche weg) versetzte Abstützung und ein Andruckelement aufweist, welches in der Messposition zwischen der Abstützung und dem Testfeldbereich gegen die zweite Seite des Teststreifens drückt, so dass das Testelement in der Messposition unter Biegespannung steht, wodurch der definierte Abstand des mindestens einen Testfeldes von der Messeinheit sichergestellt wird. Derartige Positioniereinrichtungen sind in der Anmeldung EP-A-1997112668 beschrieben.

Eine Biegung des Testelements um eine quer zu seiner Längsachse und parallel zu seiner Oberfläche orientierte Biegeachse kann für die Positionierung genutzt werden. Die Biegespannung entsteht dabei durch die Elastizität der Basisschicht des Testelements. Als Testelement im Sinne der Erfindung sollen daher alle Analyseelemente (Testträger) verstanden werden, die aufgrund ihrer Materialeigenschaften und Dimensionen eine ausreichende Elastizität für eine derartige Positionierung haben.

In einer Ausführungsform weist das Testelement in seinem Vorderabschnitt eine Ausnehmung auf. Die Aufnahmefläche weist an entsprechender Stelle im Vorderabschnitt einen federnd gelagerten Haltestift auf. Der Haltestift wird vorzugsweise von einer abnehmbaren oder schwenk- oder klappbaren Kappe überdeckt. Beim Positionierungsvorgang rastet der Haltestift in der vorderen Ausnehmung des Testelements ein. Bevorzugt weist das Testelement eine zweite Ausnehmung im Testfeldbereich auf, die mit einer Positioniereinrichtung an der Messöffnung der Aufnahmefläche des Gehäuses der Messvorrichtung übereinstimmt. Bevorzugt ist diese Positioniereinrichtung ein erhabener Rand um die Messöffnung. Durch ein Einhaken der Positioniereinrichtung in die Ausnehmung wird eine Links-rechts-Verschiebung des Testelements verhindert. Alternativ oder zusätzlich kann die Messvorrichtung Führungsschienen im Testfeldbereich oder im Handhabungsbereich aufweisen.

Bei dem erfindungsgemäßen Analysesystem wird eine Positionierung und Fixierung des Testelements bevorzugt in der vordefinierten Messposition alleine durch die Einführbewegung der Testelemente in die Halterung ermöglicht. In einer bevorzugten Ausführungsform muss weder durch den Benutzer noch durch einen Antrieb des Auswertegerätes eine Mechanik zur Fixierung der Testelemente im Auswertegerät betätigt werden.

Die exakte Positionierung des Testfeldes wird bevorzugt ohne irgendwelche in der Nähe des Testfeldbereiches von oben auf das Testelement drückende Teile erreicht.

In einer bevorzugten Ausführungsform weist die Aufnahmefläche im Bereich des Vorderendes eine erste Positioniereinrichtung und im Bereich der Messöffnung eine zweite Positioniereinrichtung auf. Die erste Positioniereinrichtung kann drehbar gelagerte konische Nocken, ein Spannstift, ein Haltedorn, ein federnd gelagerter Haltestift oder ähnliches sein, wobei das Testelement eine entsprechend ausgebildete Ausnehmung im Vorderabschnitt aufweist. In einer besonderen Ausführungsform ist die erste Positioniereinrichtung durch eine Abdeckung abgedeckt. Die Abdeckung kann auch Teil der ersten Positioniereinrichtung sein, d. h. sie kann selbst der Positionierung des Testelements dienen. Die zweite Positioniereinrichtung sind in einer bevorzugten Ausgestaltung Führungselemente. In einer anderen ebenfalls bevorzugten Ausgestaltung ist die Aufnahmefläche zumindest im Bereich der Messöffnung schmaler als das in der Messposition positionierte Testelement. Bevorzugt dient ein erhabener Rand der Messöffnung, der in das Loch der Bodenfolie des Testelements eingreifen kann, als zweite Positioniereinrichtung.

Um die Aufnahmefläche, insbesondere die Messöffnung und/oder eine Positioniereinrichtung bei der Lagerung oder beim Transport der Messvorrichtung vor Verschmutzungen und ähnlichem zu schützen, kann die Messvorrichtung eine Abdeckung aufweisen. Die Abdeckung kann klappbar, schiebbar, schraubbar oder steckbar ausgebildet sein. Die Abdeckung kann als Standfläche der Messvorrichtung bei Nichtgebrauch ausgebildet sein.

Die Erfindung wird im Folgenden anhand von in den Figuren schematisch dargestellten Ausführungsbeispielen näher erläutert; es zeigen:
Fig. 1 Eine perspektivische schematische Darstellung einer erfindungsgemäßen Messvorrichtung.
Fig. 2 Eine perspektivische schematische Darstellung einer weiteren erfindungsgemäßen Messvorrichtung.
Fig. 3 Eine perspektivische schematische Darstellung einer erfindungsgemäßen Messvorrichtung einer weiteren Ausgestaltung.
Fig. 4 Eine perspektivische schematische Darstellung einer erfindungsgemäßen Messvorrichtung einer weiteren Ausgestaltung.
Fig. 5 Eine perspektivische schematische Darstellung einer erfindungsgemäßen Messvorrichtung einer weiteren Ausgestaltung.
Fig. 6 Eine perspektivische schematische Darstellung einer erfindungsgemäßen Messvorrichtung einer weiteren Ausgestaltung.
Fig. 7 Eine perspektivische schematische Darstellung einer erfindungsgemäßen Messvorrichtung einer weiteren Ausgestaltung.
Fig. 8 Eine perspektivische schematische Darstellung einer erfindungsgemäßen Messvorrichtung einer weiteren Ausgestaltung.
Fig. 9 Eine perspektivische schematische Darstellung einer erfindungsgemäßen Messvorrichtung einer weiteren Ausgestaltung.
Fig. 10 Eine perspektivische schematische Darstellung einer erfindungsgemäßen Messvorrichtung einer weiteren Ausgestaltung.
Fig. 11 Eine perspektivische schematische Darstellung einer erfindungsgemäßen Messvorrichtung einer weiteren Ausgestaltung.
Fig. 12 Eine perspektivische Darstellung einer erfindungsgemäßen Messvorrichtung in einer bevorzugten Ausgestaltung.
Fig. 13 Eine perspektivische Darstellung einer erfindungsgemäßen Messvorrichtung in einer bevorzugten Ausgestaltung mit einer bevorzugten Positioniereinrichtung.
Fig. 14 Eine perspektivische Darstellung einer erfindungsgemäßen Messvorrichtung in einer bevorzugten Ausgestaltung mit einer ebenfalls bevorzugten Positioniereinrichtung.
Fig. 15 Eine perspektivische Darstellung einer ebenfalls erfindungsgemäßen Messvorrichtung in einer bevorzugten Ausgestaltung mit einer Positioniereinrichtung.

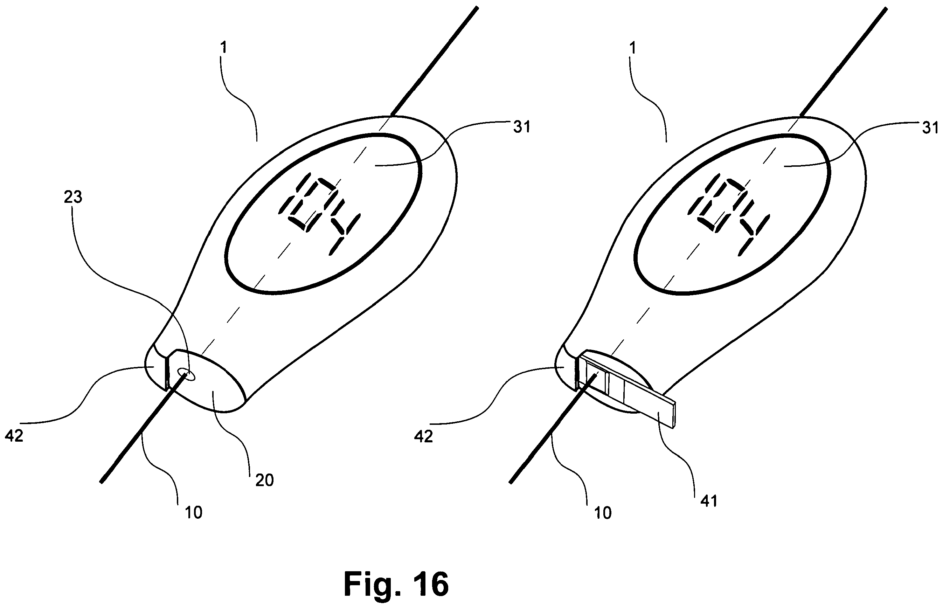

Fig. 16 Eine perspektivische Darstellung einer erfindungsgemäßen Messvorrichtung in einer bevorzugten Ausgestaltung mit und ohne Testelement.
Fig. 17 Eine perspektivische Darstellung einer erfindungsgemäßen Messvorrichtung in einer Ausgestaltung mit einer bevorzugten Positioniereinrichtung.
Fig. 18 Eine bevorzugten Positioniereinrichtung.
Fig. 19 Eine bevorzugten Positioniereinrichtung.
Fig. 20 Eine perspektivische Darstellung einer erfindungsgemäßen Messvorrichtung in einer bevorzugten Ausgestaltung mit einer bevorzugten Positioniereinrichtung.
Fig. 21 Eine bevorzugte Ausgestaltung der Aufnahmefläche.
Fig. 22 Eine bevorzugten Positioniereinrichtung.
Fig. 23 Dieselbe Positioniereinrichtung mit und ohne Kappe.

### Bezugszeichen

- 1: Gehäuse
- 10: Hauptgeräteachse
- 11: zweite Geräteachse
- 12: Geräteebene
- 20: Aufnahmefläche
- 21: Längsachse der Aufnahmefläche
- 22: durch die Aufnahmefläche aufgespannte Ebene
- 23: Messöffnung
- 31: Display auf der Frontseite der Messvorrichtung
- 41: Testelement
- 42: Positioniereinrichtung
- 43: Richtung der Positionierung

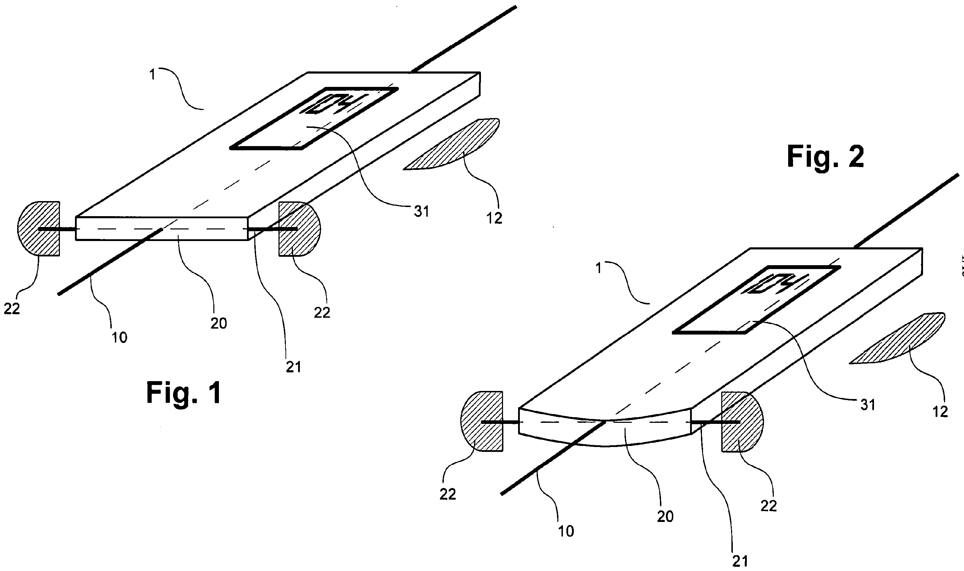

Die Figuren 1 bis 11 zeigen Ausführungsformen, bei denen das Gehäuse der Messvorrichtung näherungsweise die geometrische Form eines geraden Zylinders mit einer rechteckigen Grund- und Deckfläche hat. Diese Form dient der Erläuterung der Erfindung. Bevorzugte geometrische Formen der Grund- und Deckfläche führen zu einem ergonomisch geformten Gehäuse.

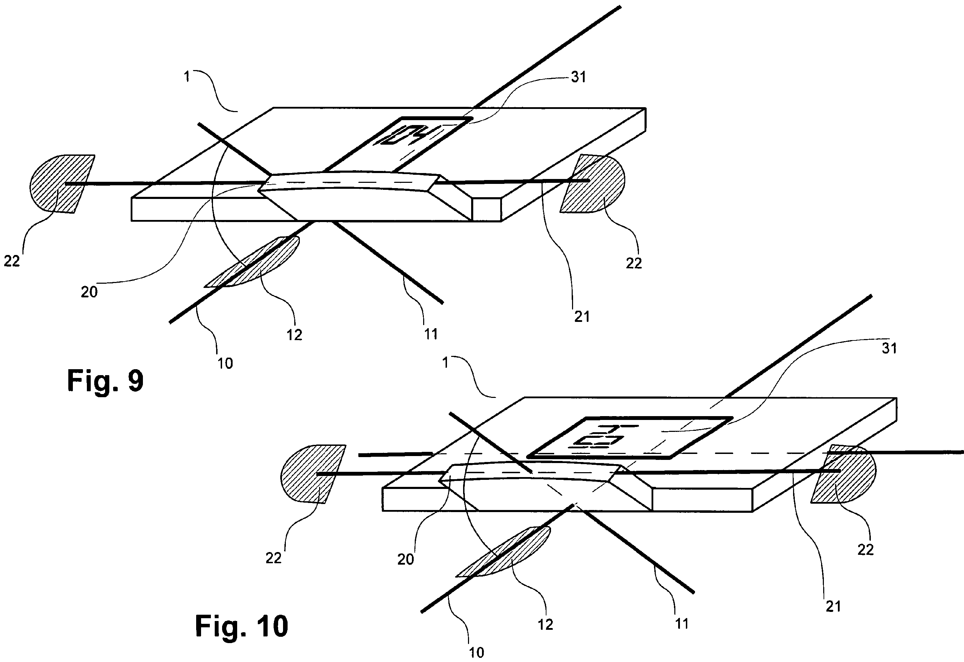

Die Figuren 1, 2 und 10 zeigen Ausführungsformen, bei denen die gesamte Schmalseite des Gehäuses **1,** auf der die Aufnahmefläche **20** angeordnet ist, die Aufnahmefläche **20** bildet. Die Achse **21** der Aufnahmefläche **20** schließt mit der Hauptgeräteachse **10** einen Winkel von 90° ein. Die Ebene **22,** die durch die Aufnahmefläche **20** aufgespannt wird, ist in den Figuren 1 und 10 senkrecht zu der Geräteebene **12** und in der Figur 2 um 45° um die Achse der Aufnahmefläche **20** gedreht. In der Figur 2 ist die Aufnahmefläche **20** gegenüber der Ebene **22,** die durch die Aufnahmefläche **20** aufgespannt wird, gekrümmt.

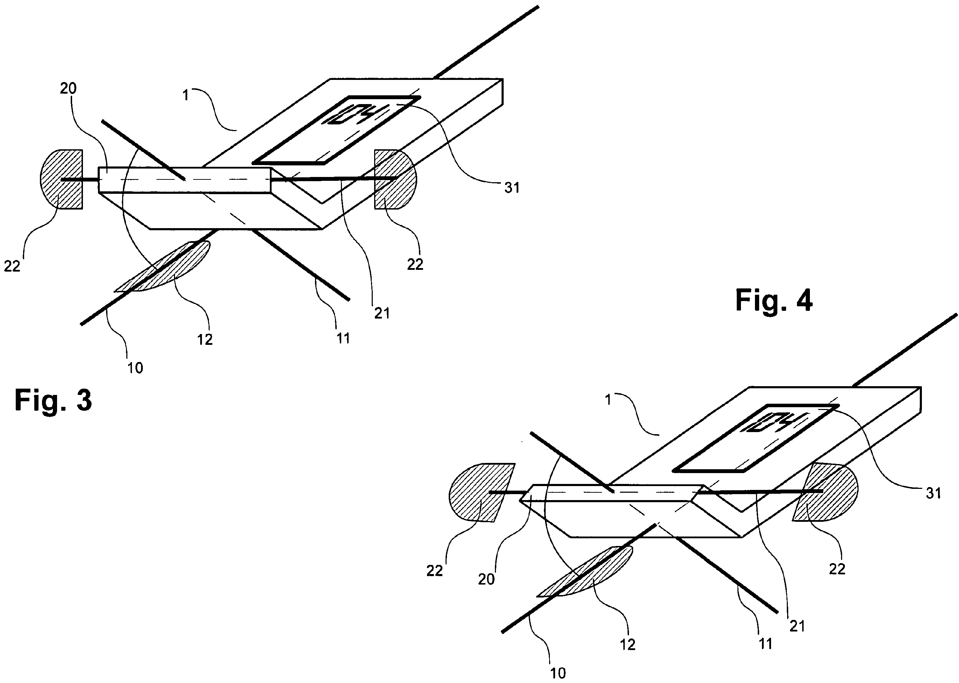

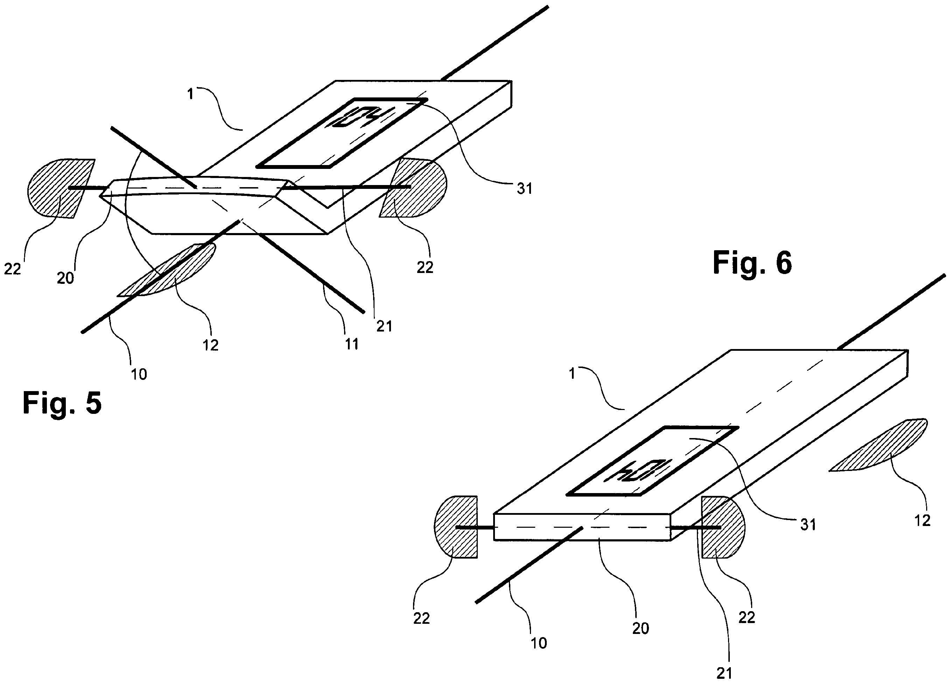

Die Figuren 3, 4 und 5 zeigen Ausführungsformen, bei denen die gesamte Schmalseite des Gehäuses **1,** auf der die Aufnahmefläche **20** angeordnet ist, die Aufnahmefläche **20** bildet. Derjenige Gehäuseteil, auf der die Aufnahmefläche **20** angeordnet ist, ist gegenüber dem übrigen Gehäuse **1** abgewinkelt. Die zweite Geräteachse **11** liegt in der gleichen die Frontseite der Messvorrichtung gliedernde Ebene wie die Hauptgeräteachse **10.** Sie schließt mit der Geräteebene **12** einen Winkel von 60° ein. Die Achse **21** der Aufnahmefläche **20** schließt mit der zweiten Geräteachse **11,** die in der gleichen, die Frontseite der Messvorrichtung gliedernde Ebene wie die Hauptgeräteachse **10** liegt, einen Winkel von 90° ein. Die Ebene **22,** die durch die Aufnahmefläche **20** aufgespannt wird, ist in der Figur 3 senkrecht zu der Geräteebene **12** und schließt in den Figuren 4 und 5 mit der Geräteebene **12** einen Winkel von 45° ein. In der Figur 5 ist die Aufnahmefläche **20** gegenüber der Ebene **22,** die durch die Aufnahmefläche **20** aufgespannt wird, gekrümmt.

Die Figuren 6, 7, 8 und 9 zeigen Ausführungsformen, bei denen lediglich ein Abschnitt Schmalseite des Gehäuses **1,** auf der die Aufnahmefläche **20** angeordnet ist, die Aufnahmefläche **20** bildet. Dieser Abschnitt weist eine exponierte Lage auf. Die Figuren 6, 7 und 8 zeigen Ausführungsformen, bei denen derjenige Gehäuseteil, auf der die Aufnahmefläche **20** angeordnet ist, gegenüber dem übrigen Gehäuse **1** abgewinkelt ist. Die zweite Geräteachse **11** schließt mit der Geräteebene **12** einen Winkel von 60° ein.

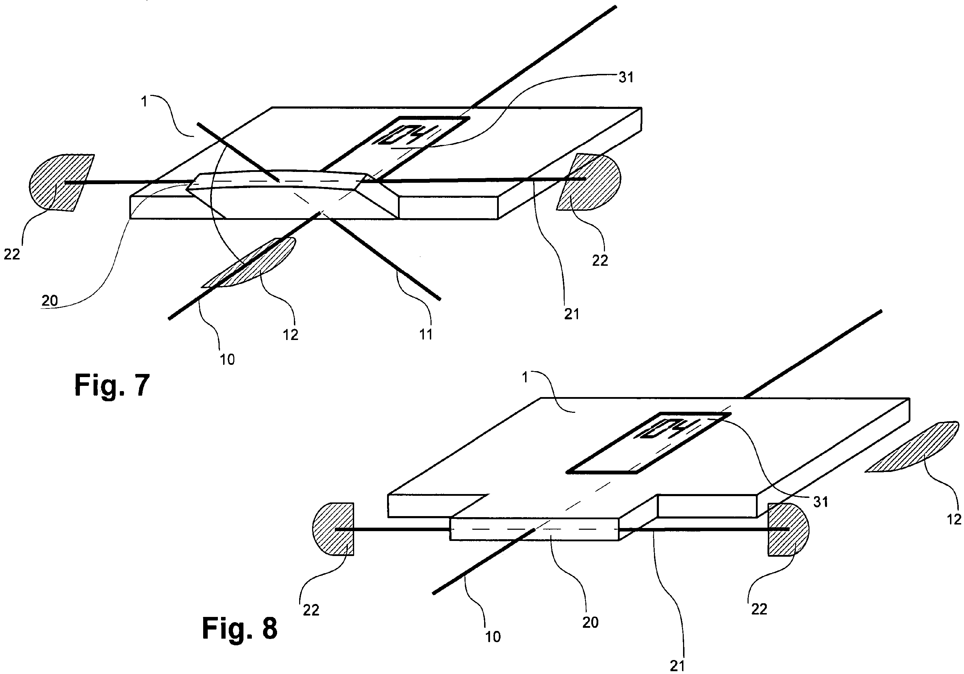

Die zweite Geräteachse **11** liegt in den Figuren 7 und 8 in der gleichen die Frontseite der Messvorrichtung gliedernde Ebene wie die Hauptgeräteachse **10.** Die Achse **21** der Aufnahmefläche **20** schließt mit der zweiten Geräteachse **11** einen Winkel von 90° ein. In der Figur 6 ist die zweite Geräteachse **11** senkrecht zu der Ebene, die die Frontseite der Messvorrichtung gliedert. Die Achse **21** der Aufnahmefläche **20** ist parallel zu der Hauptgeräteachse **10.** In den Figuren 6 bis 8 ist die Aufnahmefläche **20** gegenüber der Ebene **22,** die durch die Aufnahmefläche **20** aufgespannt wird, gekrümmt.

Die Ebene **22,** die durch die Aufnahmefläche **20** aufgespannt wird, ist in der Figur 9 senkrecht zu der Geräteebene **12** und schließt in den Figuren 6 bis 8 mit der Geräteebene **12** einen Winkel von 45° ein.

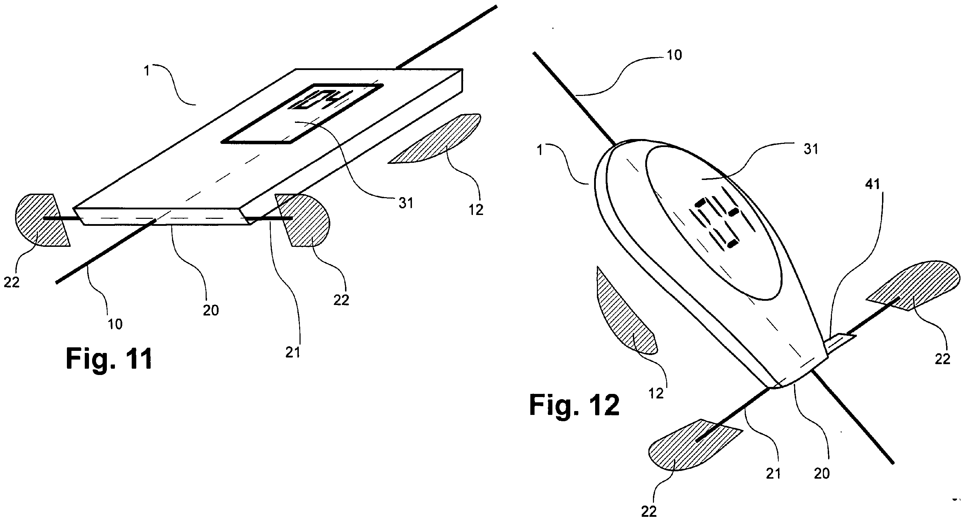

Die Figur 11 zeigt eine Ausführungsform, bei der die gesamte Schmalseite des Gehäuses **1,** auf der die Aufnahmefläche **20** angeordnet ist, die Aufnahmefläche **20** bildet. Die Achse **21** der Aufnahmefläche **20** schließt mit der Hauptgeräteachse **10** einen Winkel von 90° ein. Die Ebene **22,** die durch die Aufnahmefläche **20** aufgespannt wird, ist um -25° um die Achse der Aufnahmefläche **20** gedreht.

In Figur 12 wird eine Ausführungsform gezeigt, bei der die Grund- und Deckfläche eine elliptische Form in Kombination mit einem Rechteck aufweisen. Die Kanten, die Übergangsecken vom Rechteck zu Ellipse der Grundfläche mit der Deckfläche verbinden, sind stark abgerundet, sodass das Gehäuse **1** zwei Schmalseiten aufweist. Die gesamte Schmalseite des Gehäuses **1,** auf der die Aufnahmefläche **20** angeordnet ist, bildet dabei die Aufnahmefläche **20.** Die Achse **21** der Aufnahmefläche **20** schließt mit der Hauptgeräteachse **10** einen Winkel von 90° ein. Die Deckfläche ist konvex, die Grundfläche parallel dazu konkav gekrümmt. Die Ebene **22,** die durch die Aufnahmefläche **20** aufgespannt wird, ist um -20° um die Achse der Aufnahmefläche **20** gedreht.

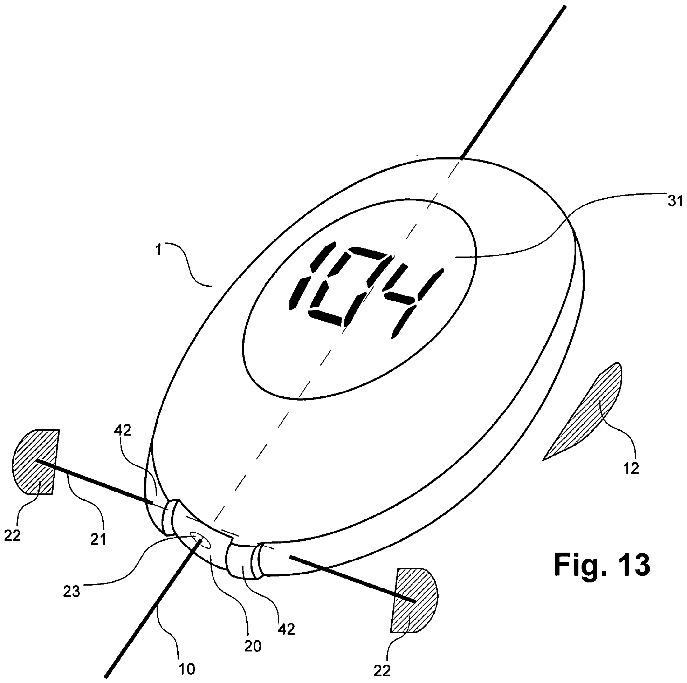

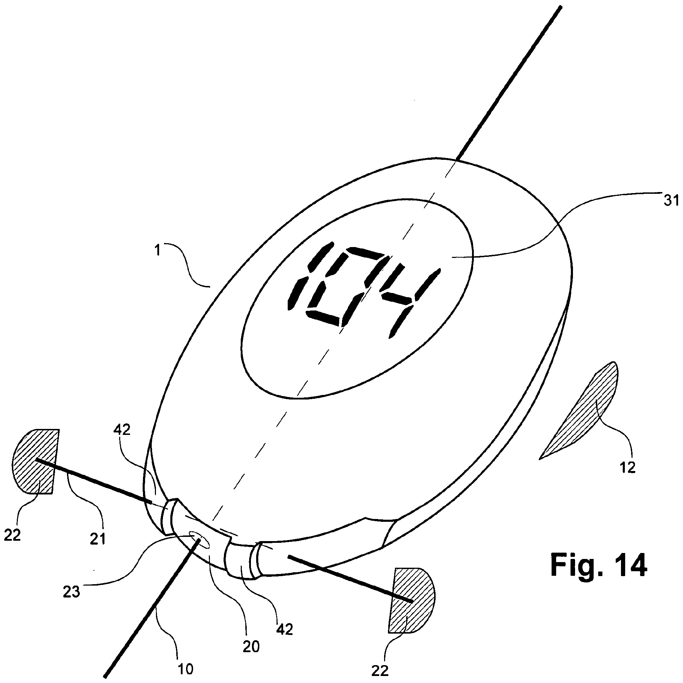

Die in Figur 13 gezeigte Ausführungsform weist eine Grund- und Deckfläche mit elliptischer Form auf, sodass das Gehäuse **1** eine Schmalseite aufweist. Auf einem Abschnitt der Schmalseite des Gehäuses **1** ist die Aufnahmefläche **20** angeordnet. Die Achse **21** der Aufnahmefläche **20** schließt mit der Hauptgeräteachse **10** einen Winkel von 90° ein. Die Deckfläche ist konvex gekrümmt. Die Positioniereinrichtungen **42** sind als beidseitige Führungsschienen ausgebildet, die integral gebildet sind und die Aufnahmefläche überdecken. Sie sind im Vorderabschnitt und im Handhabungsabschnitt angeordnet. Die in Figur 14 gezeigte Ausführungsform entspricht der in Figur 13, wobei die Kanten zwischen Schmalseite und Grund- und Deckfläche außerhalb des Bereichs der Schmalseite, die die Aufnahmefläche **20** bildet, abgerundet ist, so dass sie zu einer einzigen Kante vereinigt sind.

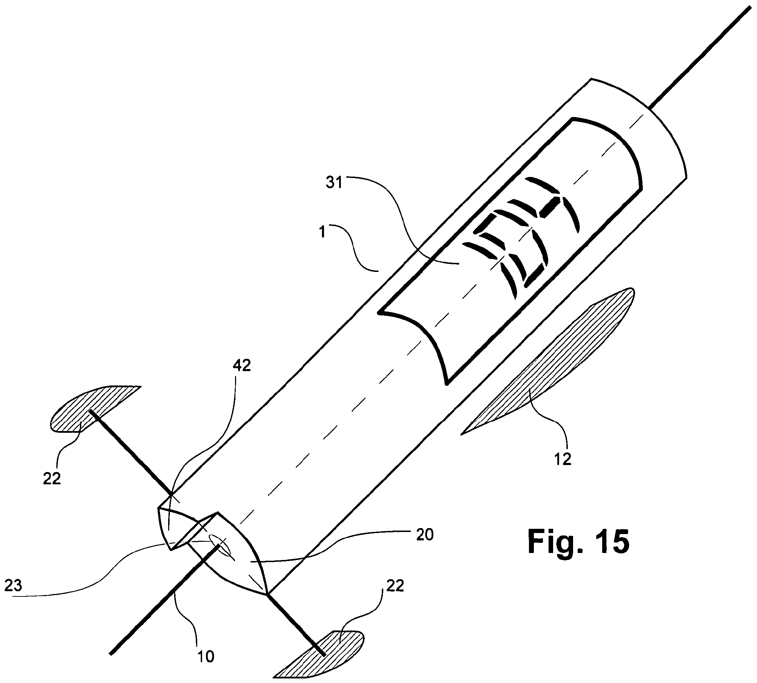

Die in Figur 15 gezeigte Ausführungsform weist eine Grund- und Deckfläche mit rechteckiger Form auf. Die Kanten zwischen beiden längsseitigen Schmalseiten und Grund- und Deckfläche sind stark abgerundet, so dass sie jeweils zu einer einzigen Kante vereinigt sind. Das Gehäuse **1** zwei Schmalseiten aufweist. Eine dieser Schmalseiten des Gehäuses 1 bildet die Aufnahmefläche **20.** Die Ebene **22,** die durch die Aufnahmefläche **20** aufgespannt wird, ist um -10° um die Achse **21** der Aufnahmefläche **20** gedreht. Die Deckfläche und die Grundfläche sind konvex gekrümmt. Die Achse **21** Aufnahmefläche **20** schließt mit der Hauptgeräteachse **10** einen Winkel von 90° ein. Die Positioniereinrichtungen **42** sind als beidseitige Führungsschienen ausgebildet, die integral gebildet sind und die Aufnahmefläche überdecken. Sie sind im Vorderabschnitt und im Handhabungsabschnitt angeordnet.

Figur 12 zeigt eine Ausführungsform, bei der die Grund- und Deckfläche eine elliptische Form in Kombination mit einem Rechteck aufweisen. Die Kanten, die Übergangsecken vom Rechteck zu Ellipse der Grundfläche mit der Deckfläche verbinden, sind stark abgerundet, sodass das Gehäuse **1** zwei Schmalseiten aufweist. Die Kanten zwischen der Schmalseite und Grund- und Deckfläche außerhalb des Bereichs der Schmalseite, die die Aufnahmefläche **20** bildet, sind derart abgerundet, dass sie einen kantenlosen Übergang von der Grundfläche zur Deckfläche bilden. Die gesamte Schmalseite des Gehäuses **1,** auf der die Aufnahmefläche **20** angeordnet ist, bildet dabei die Aufnahmefläche **20.** Die Achse **21** (nicht gezeigt) der Aufnahmefläche **20** schließt mit der Hauptgeräteachse **10** einen Winkel von 90° ein. Die Ebene **22** (nicht gezeigt), die durch die Aufnahmefläche **20** aufgespannt wird, steht senkrecht zu der Geräteebene **12** (nicht gezeigt). Die Ausführungsform ist links ohne Testelement **41** gezeigt, so dass die Messöffnung **23** sichtbar ist. Rechts ist die gleiche Ausführungsform mit Testelement **41** gezeigt.

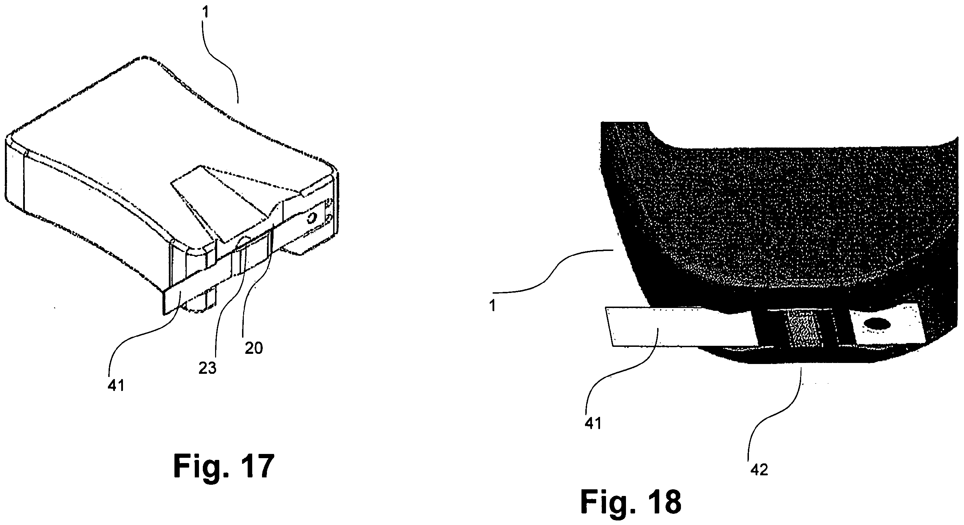

Figur 17 zeigt eine Ausführungsform, bei der die Grund- und Deckfläche eine rechteckige Form aufweisen. Die Kanten, die Ecken vom Rechteck der Grundfläche mit der Deckfläche verbinden, sind abgerundet. Das Gehäuse **1** weist vier Schmalseiten auf. Die gesamte Schmalseite des Gehäuses **1,** auf der die Aufnahmefläche **20** angeordnet ist, bildet dabei die Aufnahmefläche **20.** Die Achse **21** (nicht gezeigt) der Aufnahmefläche **20** schließt mit der Hauptgeräteachse **10** einen Winkel von 90° ein. Die Ebene **22** (nicht gezeigt), die durch die Aufnahmefläche **20** aufgespannt wird, steht senkrecht zu der Geräteebene **12** (nicht gezeigt). Die Ausführungsform ist mit Testelement **41** gezeigt, dass noch nicht in der Positionierungsendlage liegt, so dass die Messöffnung **23** sichtbar ist. Die Aufnahmefläche **20** ist im Testfeldbereich schmaler ausgebildet als das Testelement.

Figur 18 zeigt eine Ausführungsform mit Führungsschienen als Positioniereinrichtung **42** im Testfeldbereich. Die Führungsschienen überdecken das Testelement im Bereich vor und hinter dem Testfeld teilweise, um eine vertikale Verschiebung des Testelements zu verhindern.

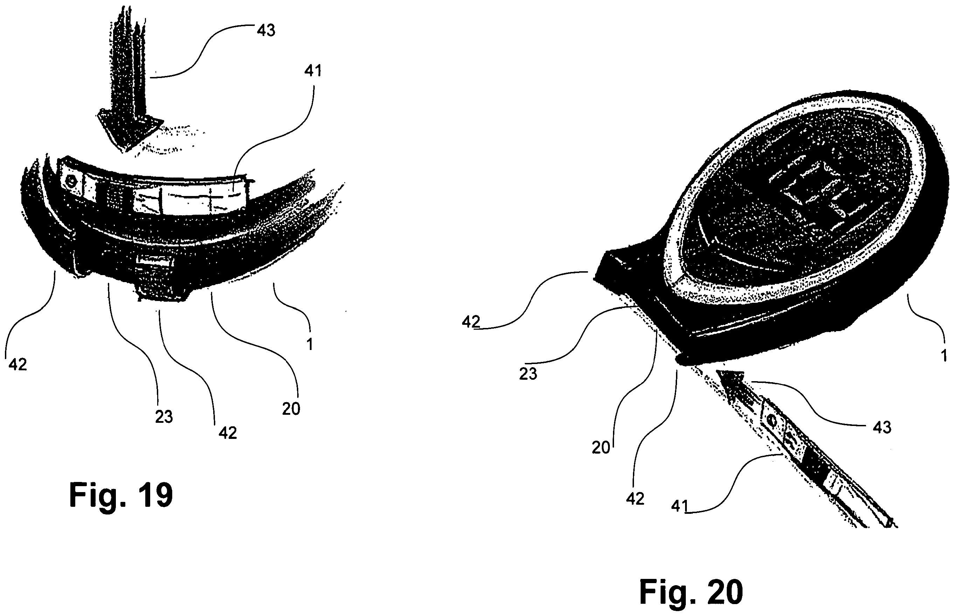

In der Figur 19 ist eine Positioniereinrichtung **42** gezeigt, die als einseitige Führungsschienen im Vorderabschnitt und im Handhabungsabschnitt ausgebildet sind. Die Führungsschienen überdecken in diesen Bereichen die Aufnahmefläche **20.** Dadurch, dass die Führungsschienen einseitig ausgebildet sind, kann der Positionierungsvorgang aus Richtung **43** der Frontseite der Messvorrichtung erfolgen.

Die Ausführungsform in Figur 20 weist zwei Positioniereinrichtungen **42** auf. Eine erste Positioniereinrichtung im Vorderabschnitt ist in Form einer beidseitigen Führungsschiene ausgebildet, die die Aufnahmefläche **20** überdeckt. Unter dieser überdeckenden Kappe ist ein Haltestift angeordnet, der mit der entsprechenden Ausnehmung eines in Messposition positionierten Testelements **43** in Eingriff steht. Eine zweite Positioniereinrichtung ist ebenfalls in Form einer beidseitigen Führungsschiene ausgebildet, die die Aufnahmefläche **20** jedoch nicht überdeckt. Die zweite Positioniereinrichtung ist am Handhabungsende angeordnet.

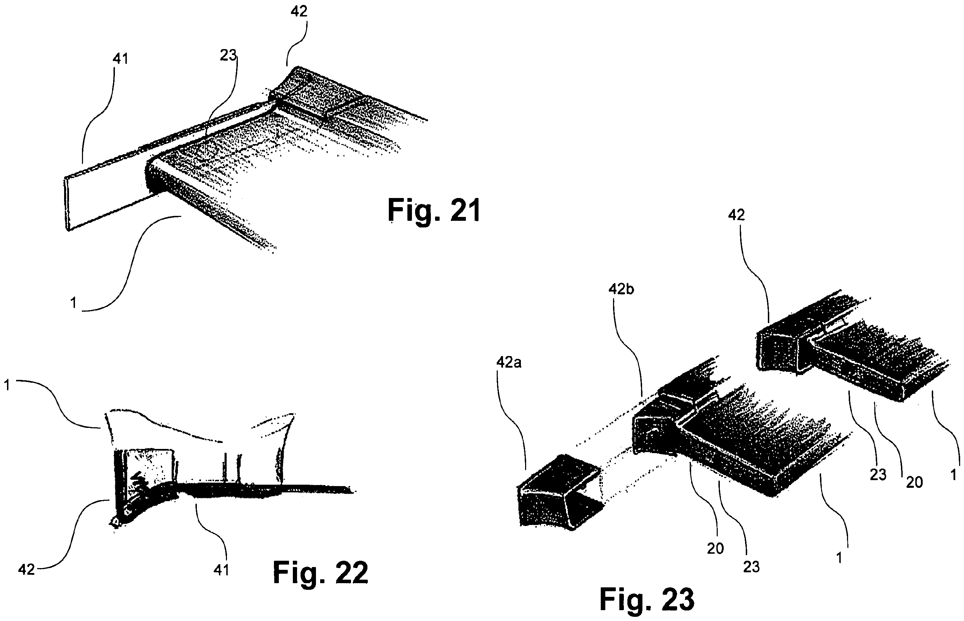

Figur 21 zeigt eine Aufnahmefläche **21,** die im Testfeldbereich und im Handhabungsabschnitt schmaler als das Testelement **41** ist. Im Bereich der Positioniereinrichtung **42** am Vorderende der Aufnahmefläche **20** ist die Aufnahmefläche **20** breiter als das Testelement **41.** Die Figur **22** zeigt eine derartige Positioniereinrichtung **42** als Schnittdarstellung. Zu erkennen ist der federnd gelagerte Haltestift. In der Figur **23** ist die abnehmbare Kappe **42a** der Positioniereinrichtung **42** sowie die Aufnahmefläche **20** mit und ohne Kappe **42a** gezeigt.

## Patentansprüche

1. Analysesystem umfassend eine tragbare diagnostische Messvorrichtung zur Bestimmung mindestens eines Analyseparameters einer Körperflüssigkeit, insbesondere zur Bestimmung einer Analytkonzentration in einer Körperflüssigkeit, und mindestens ein zur einmaligen Verwendung in der Messvorrichtung vorgesehenes Testelement (41), das als Trägerstreifen ausgestaltet ist und in Längsrichtung gesehen ein Vorderende, ein diesem gegenüberliegendes Handhabungsende und einen zwischen den Enden liegenden Testfeldbereich mit mindestens einem Testfeld aufweist, wobei die Messvorrichtung
ein Gehäuse (1) und eine Aufnahmefläche (20) zur Aufnahme des Testelements (41) umfasst, wobei das Gehäuse (1) im Wesentlichen die geometrische Form eines Zylinders mit einer Grundfläche, einer Deckfläche und einer als Schmalseiten ausgebildeten Mantelfläche aufweist, wobei die Grundfläche die Rückseite und die Deckfläche die Frontseite des Gehäuses bildet, wobei die Aufnahmefläche (20) auf einer Schmalseite angeordnet ist, wobei eine gesamte Schmalseite oder ein Abschnitt einer Schmalseite die Aufnahmefläche (20) bildet, wobei die Aufnahmefläche (20) eine Messöffnung (23) und wenigstens eine Positioniereinrichtung (42) zur Positionierung des Testelements (41) auf der Aufnahmefläche (20) aufweist, sodass das Testelement (41) flächig an der Aufnahmefläche (20) anliegt.

2. Analysesystem nach Anspruch 1, wobei die Schmalseite oder der Abschnitt der Schmalseite, die die Aufnahmefläche (20) bildet, gegenüber benachbarten Bauteilen der Messvorrichtung erhaben ist und/oder eine exponierte Lage aufweist.

3. Analysesystem nach einem der Ansprüche 1 und 2, wobei derjenige Gehäuseteil, auf dem die Aufnahmefläche (20) angeordnet ist, gegenüber dem übrigen Gehäuse (1) abgewinkelt ist, der Winkel im Bereich von 30° bis 70° vorzugsweise liegt.

4. Analysesystem nach einem der Ansprüche 1 bis 3, wobei die Ebene (22), die durch die Aufnahmefläche (20) aufgespannt wird, senkrecht zu der Frontseite ist oder gegenüber der senkrechten Anordnung um Winkel von 20° bis 70° um die Längsachse der Aufnahmefläche zur Frontseite der Messvorrichtung geneigt ist oder gegenüber der senkrechten Anordnung um Winkel von 10° bis 25° um die Längsachse der Aufnahmefläche von der Frontseite der Messvorrichtung weg geneigt ist.

5. Analysesystem nach einem der Ansprüche 1 bis 4, wobei die Messvorrichtung unterhalb der Messöffnung (23) eine Messeinheit, vorzugsweise eine optische Messeinheit aufweist.

6. Analysesystem nach einem der Ansprüche 1 bis 5, wobei die Aufnahmefläche (20) gegenüber der Ebene (22), die durch die Aufnahmefläche (20) aufgespannt wird, gekrümmt ist.

7. Analysesystem nach einem der Ansprüche 1 bis 6, wobei die Aufnahmefläche (20) mehr als eine Positioniereinrichtung (42), vorzugsweise zwei oder drei Positioniereinrichtungen (42) aufweist.

8. Analysesystem nach einem der Ansprüche 1 bis 7, wobei die wenigstens eine Positioniereinrichtung (42) ausgestaltet ist als
- in Längsrichtung der Aufnahmefläche (20) einseitig oder beidseitig angeordnete Führungselemente und/oder
- ein federnd gelagerter Haltestift, der gegebenenfalls unter einer abnehmbaren oder schwenkbaren oder klappbaren Kappe angeordnet ist, und/oder
- ein erhabener Rand einer Messöffnung (23) in der Aufnahmefläche (20).

9. Analysesystem nach einem der Ansprüche 1 bis 8, wobei mindestens ein auf dem Trägerstreifen angeordnetes Testfeld eine Fläche zum Auftrag der Körperflüssigkeit aufweist.

10. Analysesystem nach einem der Ansprüche 1 bis 9, wobei das mindestens eine Testelement (41) zumindest im Bereich eines auf dem Trägerstreifen angeordneten Testfeldes, das eine Fläche zum Auftrag der Körperflüssigkeit aufweist, breiter ist als die Aufnahmefläche der Messvorrichtung.

## Claims

1. Analysis system comprising a portable diagnostic measuring device for the determination of at least one analysis parameter of a body fluid, in particular for the determination of an analyte concentration in a body fluid, and at least one test element (41) provided for single use in the measuring device, which is designed as a carrier strip and, viewed in the longitudinal direction, has a front end, a handling end opposite thereto and a test field region with at least one test field lying between the ends, wherein the measuring device comprises a housing (1) and a receiving surface (20) for receiving the test element (41), wherein the housing (1) has substantially the geometric shape of a cylinder with a base surface, a top surface and a lateral surface formed as narrow sides, wherein the base surface forms the back and the top surface forms the front of the housing, wherein the receiving surface (20) is disposed on a narrow side, wherein an entire narrow side or a section of a narrow side forms the receiving surface (20), wherein the receiving surface (20) has a measuring opening (23) and at least one positioning device (42) for positioning the test element (41) on the receiving surface (20), such that the test element (41) lies in a planar manner on the receiving surface (20).

2. Analytical system according to claim 1, wherein the narrow side or the section of the narrow side which forms the receiving surface (20) is raised relative to neighbouring components of the measurement device and/or has an exposed position.

3. Analytical system according to one of the claims 1 and 2, wherein the housing member on which the receiving surface (20) is disposed is angled relative to the remaining housing (1) and the angle preferably lies in the range of 30° to 70°.

4. Analytical system according to one of the claims 1 to 3, wherein the plane (22) which is spanned by the receiving surface (20) is perpendicular to the front or is tilted relative to the perpendicular arrangement by an angle of 20° to 70° about the longitudinal axis of the receiving surface towards the front of the measurement device or is tilted away from the front of the measurement device relative to the perpendicular arrangement by an angle of 10° to 25° about the longitudinal axis of the receiving surface.

5. Analytical system according to one of the claims 1 to 4, wherein the measurement device has a measuring unit below the measuring opening (23), preferably an optical measuring unit.

6. Analytical system according to one of the claims 1 to 5, wherein the receiving surface (20) is curved relative to the plane (22) which is spanned by the receiving surface (20).

7. Analytical system according to one of the claims 1 to 6, wherein the receiving surface (20) has more than one positioning device (42), preferably two or three positioning devices (42).

8. Analytical system according to one of the claims 1 to 7, wherein the at least one positioning device (42) is designed as
- guide elements arranged on one side or both sides in the longitudinal direction of the receiving surface (20) and/or
- a spring-loaded holding pin which is optionally arranged under a removable or pivoted or foldable cap and/or
- a raised rim of a measuring opening (23) in the receiving surface (20).

9. Analytical system according to one of the claims 1 to 8, wherein at least one test field disposed on the carrier strip has a surface for applying body fluid.

10. Analytical system according to one of the claims 1 to 9, wherein the at least one test element (41) is wider than the receiving surface of the measurement device at least in the area of a test field disposed on the carrier strip which has a surface for applying body fluid.

## Revendications

1. Système d'analyse comprenant un dispositif de mesure diagnostique portable permettant de déterminer au moins un paramètre d'analyse d'un fluide corporel, en particulier permettant de déterminer une concentration en analytes dans un fluide corporel, et au moins un élément de test (41) prévu pour une utilisation unique dans le dispositif de mesure, qui est configuré en tant que bandelette de support et vu dans la direction longitudinale présente une extrémité avant, une extrémité de manipulation opposée à celle-ci et un secteur de zone de test se trouvant entre les extrémités avec au moins une zone de test, dans lequel le dispositif de mesure comprend un boîtier (1) et une surface de réception (20) permettant de recevoir l'élément de test (41), dans lequel le boîtier (1) présente pour l'essentiel la forme géométrique d'un cylindre avec une surface de base, une surface de couverture et une surface d'enveloppe réalisée en tant que faces étroites, dans lequel la surface de base forme la face arrière et la surface de couverture forme la face avant du boîtier, dans lequel la surface de réception (20) est disposée sur une face étroite, dans lequel l'ensemble d'une face étroite ou une section d'une face étroite forme la surface de réception (20), dans lequel la surface de réception (20) présente une ouverture de mesure (23) et au moins un dispositif de positionnement (42) permettant de positionner l'élément de test (41) sur la surface de réception (20), de sorte que l'élément de test (41) repose sur toute sa surface au niveau de la surface de réception (20).

2. Système d'analyse selon la revendication 1, dans lequel la face étroite ou la section de la face étroite qui forme la surface de réception (20) est en relief par rapport à des composants voisins du dispositif de mesure et/ou présente une situation exposée.

3. Système d'analyse selon l'une quelconque des revendications 1 et 2, dans lequel la partie de boîtier sur laquelle est disposée la surface de réception (20) forme un angle par rapport au reste du boîtier (1), l'angle se situant de préférence dans la plage de 30° à 70°.

4. Système d'analyse selon l'une quelconque des revendications 1 à 3, dans lequel le plan (22) qui s'étend à travers la surface de réception (20) est incliné de manière perpendiculaire à la face avant ou par rapport à la disposition perpendiculaire selon un angle de 20° à 70° autour de l'axe longitudinal de la surface de réception vers la face avant du dispositif de mesure ou est incliné par rapport à la disposition perpendiculaire selon un angle de 10° à 25° autour de l'axe longitudinal de la surface de réception en s'éloignant de la face avant du dispositif de mesure.

5. Système d'analyse selon l'une quelconque des revendications 1 à 4, dans lequel le dispositif de mesure présente en dessous de l'ouverture de mesure (23) une unité de mesure, de préférence une unité de mesure optique.

6. Système d'analyse selon l'une quelconque des revendications 1 à 5, dans lequel la surface de réception (20) est courbée par rapport au plan (22) qui s'étend à travers la surface de réception (20).

7. Système d'analyse selon l'une quelconque des revendications 1 à 6, dans lequel la surface de réception (20) présente plus d'un dispositif de positionnement (42), de préférence deux ou trois dispositifs de positionnement (42).

8. Système d'analyse selon l'une quelconque des revendications 1 à 7, dans lequel l'au moins un dispositif de positionnement (42) est conçu en tant
- qu'éléments de guidage disposés d'un côté ou des deux côtés dans la direction longitudinale de la surface de réception (20) et/ou
- que cheville d'arrêt montée de manière élastique qui est disposée le cas échéant sous un cache détachable ou apte à pivoter ou pliable, et/ou
- que bord en relief d'une ouverture de mesure (23) dans la surface de réception (20).

9. Système d'analyse selon l'une quelconque des revendications 1 à 8, dans lequel au moins une zone de test disposée sur la bandelette de support présente une surface permettant de déposer le fluide corporel.

10. Système d'analyse selon l'une quelconque des revendications 1 à 9, dans lequel l'au moins un élément de test (41), au moins dans le secteur d'une zone de test disposée sur la bandelette de support, qui présente une surface permettant de déposer le fluide corporel est plus large que la surface de réception du dispositif de mesure.
